# DEMANDE DE BREVET EUROPEEN

(11) **EP 0 646 581 A1**
(43) Date de publication de la demande: **05.04.1995**
(21) Numéro de dépôt: 94402115.3
(22) Date de dépôt: 23.09.1994
(51) Int. Cl.: C07D 401/10

(54) **Dérivés de 2-(2-(tétrazol-5-yl)phényl)-1,2-dihydroquinoléine, et leur utilisation comme intermédiaires**

(30) Priorité: 04.10.1993 FR 9311772
(71) Demandeur: SYNTHELABO, F-92350 Le Plessis Robinson (FR)
(72) Inventeur: Chekroun, Isaac, F-93800 Epinay (FR); Ruiz-Montes, José, F-78200 Mantes la Jolie (FR); Rossey, Guy José, F-78960 Voisins le Bretonneux (FR)
(74) Mandataire: Thouret-Lemaitre, Elisabeth

(57) **Abrégé**

Dérivés de 2-[2-(tétrazol-5-yl)phényl]-1,2-dihydro-quinoléine répondant à la formule (1) 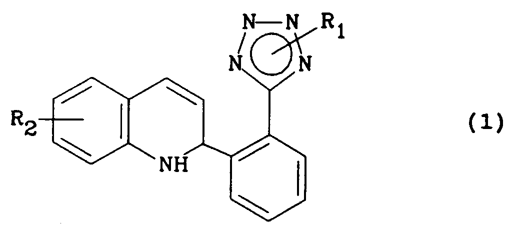
dans laquelle
R₁ représente soit un groupe (1,1-diméthyléthyle), soit un groupe triphénylméthyle, soit un groupe méthoxyméthyle, soit un groupe benzyloxyméthyle et
R₂ représente un groupe (C₁-C₄)alkyle.

Intermédiaires de synthèse.

## Description

La présente invention a pour objet des dérivés de 2-[2-(tétrazol-5-yl)phényl]-1,2-dihydroquinoléine, leur préparation et leur utilisation comme intermédiaires de synthèse.

Les composés de l'invention répondent à la formule (1) 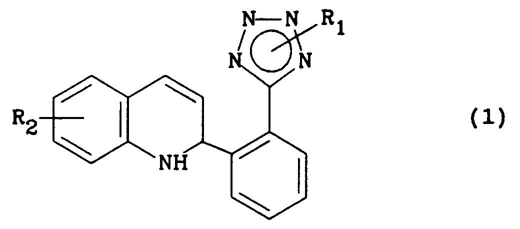
dans laquelle
R₁ représente soit un groupe (1,1-diméthyléthyle), soit un groupe triphénylméthyle, soit un groupe méthoxyméthyle, soit un groupe benzyloxyméthyle et
R₂ représente un groupe (C₁-C₄)alkyle.

Les composés préférés de l'invention sont les composés de formule (1) dans laquelle
R₁, en position 1 ou 2 sur le groupe tétrazolyle, représente soit un groupe (1,1-diméthyléthyle), soit un groupe triphénylméthyle et
R₂ est en position 6 sur la dihydroquinoléine.

Les composés selon l'invention peuvent être préparés selon le schéma 1.
On fait réagir un phényltétrazole métallé de formule (2) dans laquelle R₁ est tel que défini ci-dessus et M représente un métal choisi parmi le potassium, le lithium, le sodium et le magnésium avec une quinoléine de formule (3) dans laquelle R₂ est tel que défini ci-dessus et on obtient un composé de formule (1); la réaction a lieu dans un solvant tel que le tétrahydrofurane, le 1,2-diméthoxyéthane pur ou en mélange avec de l'hexane, du cyclohexane ou du toluène à une température comprise entre la température ambiante et la température de reflux.

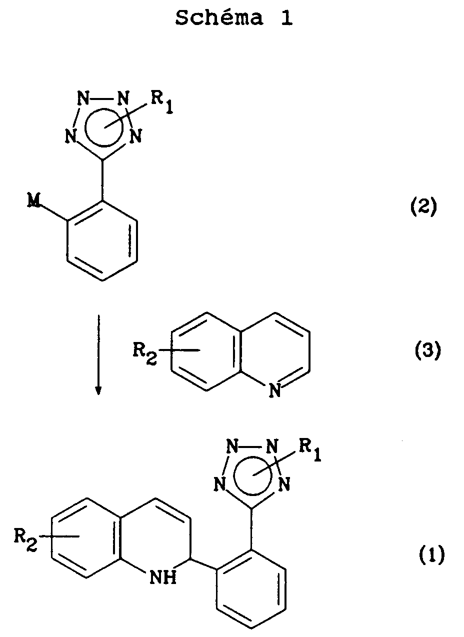

Les composés de départ sont disponibles dans le commerce ou sont décrits dans la littérature ou peuvent être préparés selon des méthodes qui y sont décrites ou qui sont connues de l'homme du métier.
Ainsi les composés de formule (2) pour lesquels M représente un atome de lithium sont préparés à partir du 5-phényltétrazole de la manière suivante : on protège le groupe tétrazolyle du 5-phényltétrazole par un groupement 1,1-diméthyléthyle selon la méthode décrite pour un dérivé analogue par J. W. TILLEY et coll. *J. Med. Chem. 1991*, **34**, 1125-1126 et on fait réagir le composé obtenu avec un alkyllithium, tel le butyllithium, dans un solvant aprotique comme le tétrahydrofurane, à une température comprise entre - 50°C et + 66 °C.

L'exemple suivant illustre l'invention. Les microanalyses et les spectres IR et RMN confirment la structure des composés obtenus.

### Exemple

2-[2-[2-(1,1-diméthyléthyl)-2*H*-tétrazol-5-yl]phényl]-6-méthyl-1,2-dihydroquinoléine
1. 2-(1,1-diméthyléthyl)-5-phényl-2*H*-tétrazole.
   Dans un ballon de 500 ml, on introduit 30 g de 5-phényltétrazole et 210 ml d'acide trifluoroacétique. A la suspension obtenue, on ajoute 10 ml d'acide sulfurique concentré et 33,6 g de 1,1-diméthyléthanol. On laisse le mélange réactionnel, sous agitation, pendant 48 heures puis on le verse sur 750 ml d'acétate d'éthyle. On lave la solution avec 2 fois 200 ml d'eau puis avec de la soude à 10 % jusqu'à pH alcalin et à nouveau avec 2 fois 200 ml d'eau. Après séchage de la phase organique et évaporation sous vide, on obtient 36,15 g de produit sous forme d'huile.
   Ce produit a les mêmes caractéristiques que celui décrit par R. A. HENRY, *J. Heterocyclic Chem., 1976*, **13**, 391-392.
2. 2-[2-[2-(1,1-diméthyléthyl)-2*H*-tétrazol-5-yl]phényl]-6-méthyl-1,2-dihydroquinoléine
   Dans un ballon tricol de 2 litres, sous atmosphère d'azote, on introduit 100 g (490 mmoles) de 2-(1,1-diméthyléthyl)-5-phényl-2*H*-tétrazole et 500 ml de tétrahydrofurane anhydre. Au mélange on ajoute goutte à goutte 215 ml (580 mmoles) d'une solution 2,5 M de n-butyllithium dans l'hexane. On chauffe le mélange à la température de reflux pendant 45 minutes puis on ajoute 49 g (410 mmoles) de 6-méthylquinoléine en solution dans 300 ml de toluène. On laisse le milieu réactionnel pendant 2 heures à la température de reflux puis on le refroidit à la température ambiante. Ensuite on ajoute 300 ml d'éthanol et on évapore sous pression réduite. On reprend le résidu dans 500 ml d'eau, on le filtre, on le lave avec 200 ml d'isopropanol et on le sèche sous vide.
   On obtient 132,5 g de produit sous forme de cristaux jaune clair que l'on utilise tel quel.
   Point de fusion = 164-166 °C

Les composés de l'invention sont particulièrement utiles pour la synthèse de composés de formule (I) 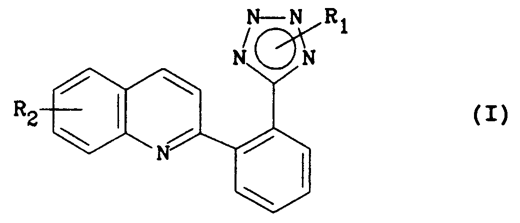
dans laquelle R₁ et R₂ sont tels que définis ci-dessus. Les composés de formule (I) sont obtenus par oxydation du noyau 1,2-dihydroquinoléine des composés de formule (1). Ainsi la réaction d'oxydation peut être réalisée soit en présence de dioxyde de manganèse dans un solvant tel que le toluène ou le xylène à une température comprise entre la température ambiante et la température de reflux, soit en présence de permanganate de potassium dans un solvant tel que l'acétate d'éthyle à la température de reflux, soit en présence d'hypochlorite de sodium, soit en présence d'eau oxygénée, soit en présence de palladium sur charbon dans un solvant tel que le toluène ou le xylène à une température comprise entre 100 °C et la température de reflux, soit en présence de nitrobenzène à une température comprise entre 100 °C et 170 °C.

L'exemple A ci-dessous illustre l'utilisation des composés de formule (1) pour la synthèse des composés de formule (I).

### Exemple A

2-[2-[2-(1,1-diméthyléthyl)-2*H*-tétrazol-5-yl]phényl]-6-méthylquinoléine
***Voie a :***
   Dans un ballon tricol de 100 ml on introduit successivement 5 g (14,4 mmoles) de 2-[2-[2-(1,1-diméthyléthyl)-2*H*-tétrazol-5-yl]phényl]-6-méthyl-1,2-dihydroquinoléine et 2,51 g (28,9 mmoles) de dioxyde de manganèse dans 50 ml de toluène. On chauffe le milieu réactionnel pendant 2 heures à 80 °C puis on le laisse revenir à la température ambiante. On filtre le milieu réactionnel sur célite et on évapore à sec.
   On obtient 4,94 g de produit sous forme de cristaux blancs.
   Point de fusion = 103-105 °C
***Voie b :***
   Dans un ballon tricol de 50 ml on introduit successivement 1 g (2,9 mmoles) de 2-[2-[2-(1,1-diméthyléthyl)-2*H*-tétrazol-5-yl]phényl]-6-méthyl-1,2-dihydroquinoléine, 0,91 g (5,8 mmoles) de permanganate de potassium et 20 ml d'acétate d'éthyle. On porte le milieu réactionnel à la température de reflux et on le chauffe pendant 1 heure. On laisse refroidir le mélange à la tempé-rature ambiante et on le filtre sur célite, on le lave avec 20 ml d'une solution à 10 % de bisulfite de sodium, on le sèche sur sulfate de magnésium et on évapore à sec.
   On obtient 0,99 g de produit sous forme de cristaux blancs.
   Point de fusion = 103-105 °C
***Voie c :***
   Dans un ballon tricol de 100 ml on introduit successivement 3,5 g (10 mmoles) de 2-[2-[2-(1,1-diméthyléthyl)-2*H*-tétrazol-5-yl]phényl]-6-méthyl-1,2-dihydroquinoléine et 20 ml de nitrobenzène. On chauffe le milieu réactionnel pendant 1 heure à 170 °C puis on le laisse revenir à la température ambiante. Ensuite on dilue le mélange avec 100 ml de toluène et on le lave successivement par 50 ml d'une solution d'acide chlorhydrique à 10 % puis par 50 ml d'une solution d'acide chlorhydrique concentré. On sépare les phases, on ajuste le pH de la phase aqueuse à 12 avec une solution de soude à 30 % et on l'extrait 3 fois par 50 ml de toluène. Ensuite on rassemble les phases organiques, on les lave successivement par 15 ml d'eau puis par 15 ml d'une solution saturée de chlorure de sodium et on évapore le solvant.
   On obtient 2,95 g de produit sous forme de cristaux blancs.
   Point de fusion = 103-105 °C
***Voie d :***
   Dans un ballon tricol de 100 ml on introduit successivement 5,9 g (17 mmoles) de 2-[2-[2-(1,1-diméthyléthyl)-2*H*-tétrazol-5-yl]phényl]-6-méthyl-1,2-dihydroquinoléine, 1 g de palladium sur charbon à 5 % et 50 ml de toluène. On chauffe le mélange pendant 3 heures à 80 °C puis on le laisse revenir à la température ambiante. Ensuite on filtre le milieu réactionnel sur célite puis on évapore le solvant.
   On obtient 5,84 g de produit sous forme de cristaux blancs.
   Point de fusion = 103-105 °C
***Voie e :***
   Dans un ballon tricol de 100 ml on introduit successivement 10 g (29 mmoles) de 2-[2-[2-(1,1-diméthyléthyl)-2*H*-tétrazol-5-yl]phényl]-6-méthyl-1,2-dihydroquinoléine, 0,1 g de chlorure de benzyltriéthylammonium, 100 ml d'une solution d'hypochlorite de sodium à 48 % dans l'eau et 80 ml de toluène. On chauffe le milieu réactionnel à la température de reflux pendant 20 heures puis on le laisse refroidir à la température ambiante. On sépare les phases et on recueille la phase organique. Ensuite on lave la phase organique successivement par 50 ml d'une solution à 10 % de bisulfite de sodium dans l'eau puis par 50 ml d'une solution saturée de chlorure de sodium, on la sèche sur sulfate de magnésium et on évapore le solvant à sec.
   On obtient 9,3 g de produit sous forme de cristaux blancs.
   Point de fusion = 103-105°C

Des composés de formule (I) sont décrits dans les demandes de brevets européens EP 0540400, EP 0569013, EP 0604259 et dans le brevet français FR 9208201.
Ces composés de formule (I) sont utiles comme intermédiaires dans la synthèse de composés antagonistes de l'angiotensine II tels que ceux décrits dans les demandes de brevets européens EP 0528762, EP 0540400, EP 0569013, EP 0604259 et dans le brevet français FR 9208201.

## Revendications

1. Dérivés de 2-[2-(tétrazol-5-yl)phényl]-1,2-dihydro quinoléine répondant à la formule (1) 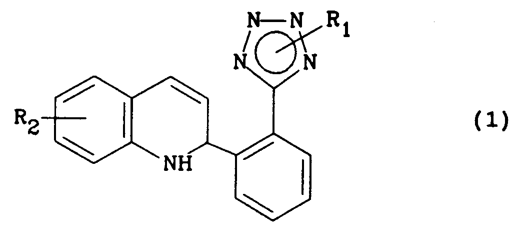 dans laquelle
R₁ représente soit un groupe (1,1-diméthyléthyle), soit un groupe triphénylméthyle, soit un groupe méthoxyméthyle, soit un groupe benzyloxyméthyle et
R₂ représente un groupe (C₁-C₄)alkyle.

2. Composés selon la revendication 1 caractérisés en ce que
R₁, en position 1 ou 2 sur le groupe tétrazolyle, représente soit un groupe (1,1-diméthyléthyle), soit un groupe triphénylméthyle et
R₂ est en position 6 sur la dihydroquinoléine.

3. La 2-[2-[2-(1,1-diméthyléthyl)-2*H*-tétrazol-5-yl]phényl]-6-méthyl-1,2-dihydroquinoléine.

4. Procédé de préparation des composés selon la revendication 1, procédé caractérisé en ce que l'on fait réagir, dans un solvant aprotique, un phényltétrazole métallé de formule (2) 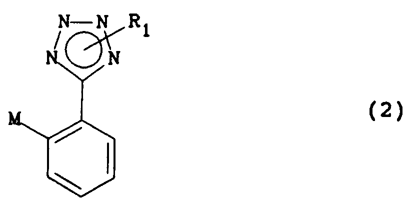 dans laquelle R₁ est tel que défini dans la revendication 1 et M représente un métal choisi parmi le potassium, le lithium, le sodium et le magnésium avec une quinoléine de formule (3) 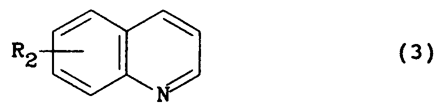 dans laquelle R₂ est tel que défini dans la revendication 1.

5. Utilisation des composés selon l'une quelconque des revendications 1 à 3 pour la synthèse des composés de formule (I) 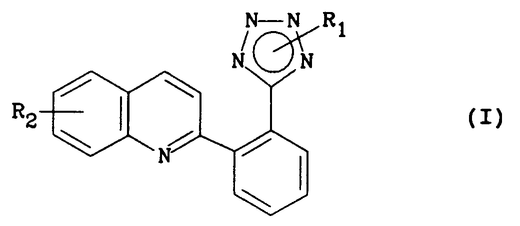 dans laquelle
R₁ représente soit un groupe (1,1-diméthyléthyle), soit un groupe triphénylméthyle, soit un groupe méthoxyméthyle, soit un groupe benzyloxyméthyle et
R₂ représente un groupe (C₁-C₄)alkyle.
